# EUROPEAN PATENT SPECIFICATION

(11) **EP 4 316 643 B1**
(45) Date of publication and mention of the grant of the patent: **10.09.2025**
(21) Application number: 22780254.3
(22) Date of filing: 18.03.2022
(51) Int. Cl.: B01J 19/12, F24F 11/52, A61L 9/20, F24F 8/22

(54) **ULTRAVIOLET LIGHT EMITTING DEVICE, AND AIR TREATING DEVICE**
ULTRAVIOLETTLICHTEMITTIERENDE VORRICHTUNG UND LUFTBEHANDLUNGSVORRICHTUNG
DISPOSITIF D'ÉMISSION DE LUMIÈRE ULTRAVIOLETTE ET DISPOSITIF DE TRAITEMENT D'AIR

(30) Priority: 31.03.2021 JP 2021060476
(43) Date of publication of application: 07.02.2024
(73) Proprietor: DAIKIN INDUSTRIES, LTD., Osaka-shi, Osaka 530-0001 (JP)
(72) Inventor: SAITO, Tomoki, Osaka-shi, Osaka 530-0001 (JP); TANAKA, Toshio, Osaka-shi, Osaka 530-0001 (JP); OKUMOTO, Mamoru, Osaka-shi, Osaka 530-0001 (JP)
(74) Representative: Hoffmann Eitle
(86) International application number: PCT/JP2022/012729
(87) International publication number: WO 2022/210039

(56) References cited:
- WO-A1-2021/202736
- WO-A1-2021/211723
- WO-A1-2022/011046
- JP-A- 2003 135 581
- JP-A- 2003 135 581
- JP-A- 2005 181 878
- JP-A- 2005 181 878
- JP-A- 2017 023 305
- JP-A- 2020 167 147
- JP-A- 2021 114 974
- KR-A- 20200 020 157
- US-A1- 2018 236 116

## Description

### TECHNICAL FIELD

The present disclosure relates to an ultraviolet light emitting device and an air treating device.

### BACKGROUND ART

An air conditioner described in Japanese Unexamined Patent Publication No. 2009-14259 includes an emitter means configured to emit ultraviolet rays into an installation space, an emission direction changing means configured to change the direction in which the ultraviolet rays are emitted, and a control means. If a moving body is present in the space, the control means controls the emission direction changing means to make the emitter means emit ultraviolet rays into the installation space while avoiding the position of the moving body based on positional information on the moving body. Thus, ultraviolet rays can be emitted into a living space while avoiding a moving body, such as a human body or an animal. Further, JP 2003 135581 A1 is concerned with an ultraviolet light emitter. KR 2020 0020157 A1 and US 2018/236116 A1 disclose UV sterilizing device comprising visible light indicators.

### SUMMARY OF THE INVENTION

### TECHNICAL PROBLEM

According to such an ultraviolet light emitting device as described in Japanese Unexamined Patent Publication No. 2009-14259, a subject cannot recognize the state of ultraviolet rays emitted from an irradiator.

It is an object of the present disclosure to provide an ultraviolet light emitting device that allows a subject to recognize the state of ultraviolet rays emitted from an irradiator.

### SOLUTION TO THE PROBLEM

A first aspect of the present disclosure is directed to an ultraviolet light emitting device as defined in claim 1.

### BRIEF DESCRIPTION OF THE DRAWINGS

FIG. 1 is a diagram schematically illustrating an ultraviolet light emitting device according to a first embodiment.
FIG. 2 is a block diagram of the ultraviolet light emitting device according to the first embodiment.
FIG. 3 is a flowchart showing operations of the ultraviolet light emitting device according to the first embodiment.
FIG. 4 is a diagram schematically illustrating an ultraviolet light emitting device according to a second embodiment, showing a state where a subject is located outside an irradiation region.
FIG. 5 is a diagram schematically illustrating the ultraviolet light emitting device according to the second embodiment, showing a state where a subject is located inside the irradiation region.
FIG. 6 is a perspective view schematically illustrating an ultraviolet light emitting device according to a third embodiment.
FIG. 7 is a diagram schematically illustrating an ultraviolet light emitting device according to a fourth embodiment.
FIG. 8 is a diagram schematically illustrating an ultraviolet light emitting device according to a fifth embodiment.
FIG. 9 is a diagram schematically illustrating an ultraviolet light emitting device according to a fifth embodiment.

### DESCRIPTION OF EMBODIMENTS

Embodiments of the present disclosure will be described in detail below with reference to the drawings. The present disclosure is not limited to the embodiments shown below,

Since each of the drawings is intended to illustrate the present disclosure conceptually, dimensions, ratios, or numbers may be exaggerated or simplified as necessary for ease of understanding.

### «First Embodiment»

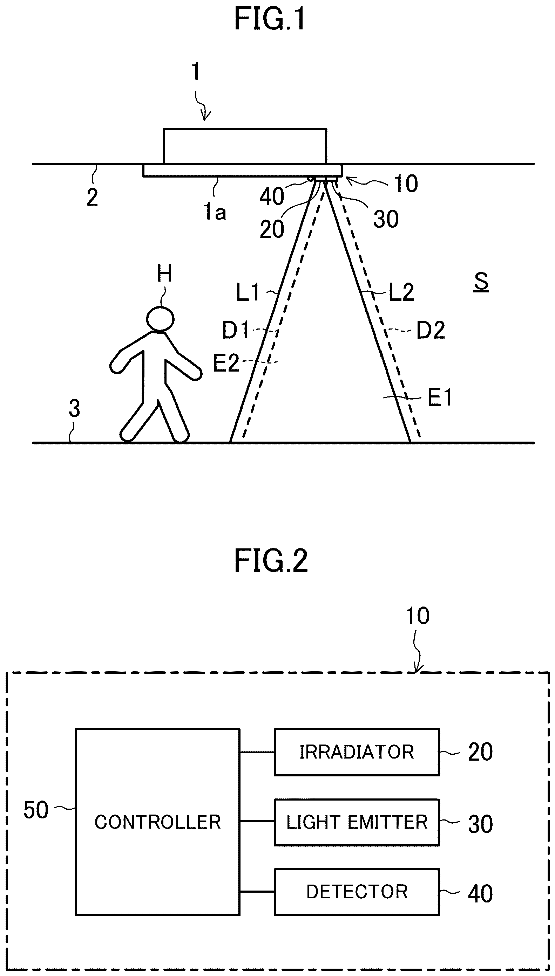

As illustrated in FIG. 1, an ultraviolet light emitting device (10) according to a first embodiment is provided for an air conditioner (1) serving as an air treating device. The air conditioner (1) is of a ceiling-mounted type. Specifically, the air conditioner (1) is of a ceiling-embedded type provided behind a ceiling (2) of a building. The air conditioner (1) may be of a ceiling suspended type. The air conditioner (1) may be of a wall-hanging type or a floor-mounted type. The air conditioner (1) includes a fan and a heat exchanger, which are not shown. The air conditioner (1) adjusts the temperature of air in a target space (S) (e.g., an indoor space).

The ultraviolet light emitting device (10) includes an irradiator (20), a light emitter (30), a detector (40), and a controller (50).

The irradiator (20) emits ultraviolet rays to the target space (S), where a subject is present. The target space (S) includes an indoor space, such as a living space, an office, or a store. The irradiator (20) includes a light source that emits ultraviolet rays. The light source includes a light source element, such as a light-emitting diode (LED) and a laser diode, an excimer lamp, and a mercury lamp. The ultraviolet rays emitted from the irradiator (20) have a wavelength of 190 nm or more and 280 nm or less. Preferably, the ultraviolet rays have a wavelength of 190 nm or more and 230 nm or less.

The irradiator (20) is provided, for example, on a lower surface of a decorative panel (1a) of the air conditioner (1). The irradiator (20) may be provided on a lower surface of the ceiling (2) so as to be adjacent to the air conditioner (1). The irradiator (20) is arranged such that the ultraviolet rays are emitted toward the floor surface (3). The irradiator (20) emits the ultraviolet rays to generate an irradiation region (E1) in the target space (S). In FIG. 1, the irradiation region (E1) is a region between the solid line L1 and the solid line L2.

The light emitter (30) is a recognizer configured to notify a person (a subject (H)) of the state of the ultraviolet rays emitted from the irradiator (20). The light emitter (30) is configured by an LED that emits visible light. The light emitter (30) is provided on the lower surface of the decorative panel (1a) of the air conditioner (1). The light emitter (30) may be provided on the lower surface of the ceiling (2) so as to be adjacent to the air conditioner (1). The light emitter (30) is arranged such that the visible light is emitted toward the floor surface (3). The light emitter (30) emits the visible light to generate a visible light region (E2) in the target space (S). In FIG. 1, the visible light region (E2) is a region between the dashed line D1 and the dashed line D2.

The irradiator (20) and the light emitter (30) are arranged adjacent to each other. The light emitter (30) emits visible light such that the visible light overlaps with the irradiation region (E1) of the ultraviolet rays emitted from the irradiator (20). Preferably, the irradiation region (E1) and the visible light region (E2) entirely coincide with each other. However, only parts of these regions may overlap with each other. The light emitter (30) of the first embodiment notifies the subject (H) of the irradiation region (E1) of the ultraviolet rays as the state of the ultraviolet rays.

The light emitter (30) is configured such that the illuminance and hue of the visible light are changeable. The light emitter (30) notifies the subject (H) of another state of the ultraviolet rays (which will be described in detail later) in accordance with the illuminance and hue of the visible light, wherein the state of the ultraviolet rays does not include an ON/OFF state of the irradiator (20).

The detector (40) detects the subject (H) in the target space (S). The detector (40) is an infrared or ultrasonic motion sensor. The detector (40) is provided, for example, on the lower surface of the decorative panel (1a) of the air conditioner (1). The detector (40) may be provided on the lower surface of the ceiling (2) so as to be adjacent to the air conditioner (1). The detector (40) detects whether or not the subject (H) is present in the target space (S).

The controller (50) shown in FIG. 2 controls the irradiator (20) and the light emitter (30). The controller (50) includes a microcomputer mounted on a control board, and a memory device (specifically, a semiconductor memory) storing software for operating the microcomputer. The controller (50) may also serve as a controller for the air conditioner (1). The controller (50) may include a server device connected to the irradiator (20) and the light emitter (30) via a network.

The controller (50) controls at least one of the intensity, irradiation amount, or wavelength of the ultraviolet rays emitted from the irradiator (20).

The controller (50) controls the light emitter (30) in accordance with the state of the ultraviolet rays emitted from the irradiator (20). The "state of the ultraviolet rays" as used herein includes the intensity of ultraviolet rays emitted from the irradiator (20), the irradiation amount of the ultraviolet rays, the wavelength of the ultraviolet rays, or the irradiation region (E1) of the ultraviolet rays. The "state of the ultraviolet rays" does not include the ON/OFF state of the irradiator (20).

The irradiation amount of ultraviolet rays [J/cm²] can be expressed by the following Equation (1). Irradiation Amount of Ultraviolet Rays [J/cm2] = Intensity of Ultraviolet Rays (Irradiation Intensity) [W/cm2] × Irradiation Time [sec]

The irradiation time [sec] is a time in which the irradiator (20) emits ultraviolet rays.

The controller (50) actuates the light emitter (30) when the detector (40) detects the subject (H). In other words, when the detector (40) detects the subject (H), the light emitter (30) serving as a recognizer notifies the subject (H) of the state of the ultraviolet rays emitted from the irradiator (20).

### -Operation of Ultraviolet Light Emitting Device-

An operation of the ultraviolet light emitting device (10) will be described.

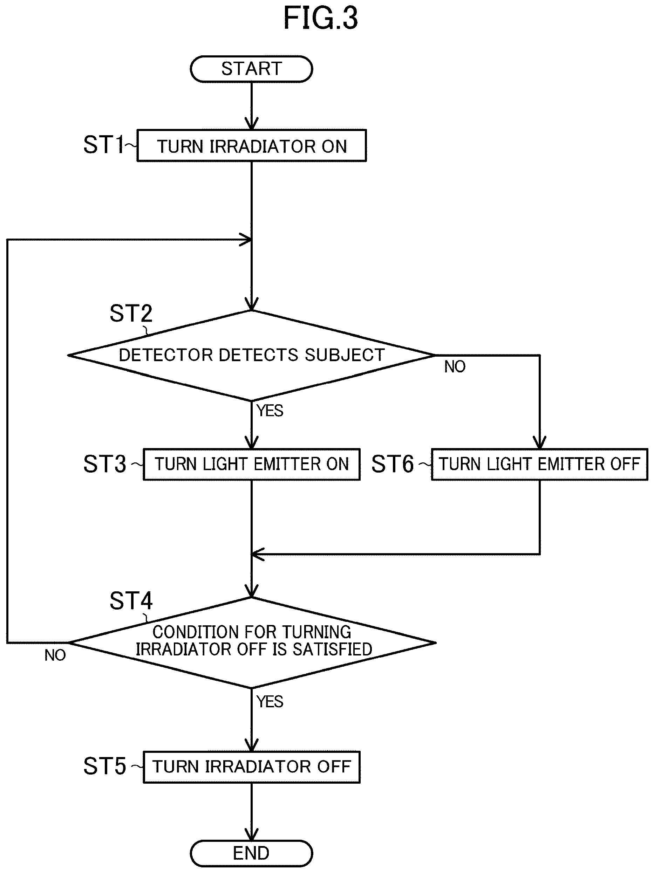

As shown in FIG. 3, the controller (50) turns the irradiator (20) on in step ST1 when the ultraviolet light emitting device (10) starts to operate. The irradiator (20) emits ultraviolet rays from the ceiling (2) toward the floor surface (3).

In step ST2, if the detector (40) detects the subject (H), step ST3 is executed. In step ST3, the controller (50) turns the light emitter (30) on in conjunction with the operation of the irradiator (20). The light emitter (30) emits visible light from the ceiling (2) toward the floor surface (3). The irradiator (20) forms the irradiation region (E1) in a predetermined range of the target space (S).

As illustrated in FIG. 1, the light emitter (30) generates the visible light region (E2) such that the visible light region (E2) overlaps with the irradiation region (E1) generated by the irradiator (20). Thus, the subject (H) can know where in the target space (S) the irradiation region (E1) of ultraviolet rays is present.

The irradiator (20) emits ultraviolet rays with a predetermined intensity when the irradiator (20) is in the on-state. The controller (50) changes the illuminance or hue of the visible light emitted from the light emitter (30) in accordance with the intensity of the ultraviolet rays emitted from the irradiator (20).

Specifically, the light emitter (30) increases the illuminance of the visible light with the increasing intensity of the ultraviolet rays and reduces the illuminance of the visible light with the decreasing intensity of the ultraviolet rays. Alternatively, the light emitter (30) makes the hue of the visible light closer to blue with the increasing intensity of the ultraviolet rays and makes the hue of the visible light closer to red or white with the decreasing intensity of the ultraviolet rays. The subject (H) can know the intensity of the ultraviolet rays emitted from the irradiator (20), based on the illuminance or hue of the visible light from the light emitter (30).

Likewise, the controller (50) may change the illuminance or hue of the visible light emitted from the light emitter (30) in accordance with the wavelength of the ultraviolet rays emitted from the irradiator (20). For example, the light emitter (30) makes the hue of the visible light closer to blue with a decreasing wavelength of the ultraviolet rays and makes the hue of the visible light closer to red or white with an increasing wavelength of the ultraviolet rays. The subject (H) can know the wavelength of the ultraviolet rays emitted from the irradiator (20), based on the illuminance or hue of the visible light from the light emitter (30).

Likewise, the controller (50) may change the illuminance or hue of the visible light emitted from the light emitter (30) in accordance with the irradiation amount of ultraviolet rays emitted from the irradiator (20). For example, the light emitter (30) increases the illuminance of the visible light with an increasing irradiation amount of the ultraviolet rays and reduces the illuminance of the visible light with a decreasing irradiation amount of the ultraviolet rays. Alternatively, the light emitter (30) makes the hue of the visible light closer to blue with an increasing irradiation amount of the ultraviolet rays emitted and makes the hue of the visible light closer to red or white with a decreasing irradiation amount of the ultraviolet rays emitted. The subject (H) can know the irradiation amount of the ultraviolet rays emitted from the irradiator (20), based on the illuminance or hue of the visible light from the light emitter (30).

As can be seen, in step ST3, the light emitter (30), which serves as a recognizer, notifies the subject (H) of the state of ultraviolet light.

If a condition for turning the irradiator (20) off is satisfied in step ST4, the controller (50) turns the irradiator (20) off in step ST5. This condition is, for example, that a predetermined time has elapsed since the irradiator (20) is turned on, or that the subject (H) has turned off the irradiator (20).

If, in step ST3, the detector (40) does not detect the subject (H) with the irradiator (20) turned on, the controller (50) turns the light emitter (30) off in step ST6.

### - Features of First Embodiment -

The ultraviolet light emitting device (10) includes the recognizer (light emitter (30)) configured to notify the subject (H) of the state of the ultraviolet light emitted from the irradiator (20) to the target space (S) where the subject is present, wherein the state of the ultraviolet rays does not include an ON/OFF state of the irradiator (20).

Thus, the subject (H) can know the state of the ultraviolet light from the irradiator (20).

The recognizer is the light emitter (30). It is thus possible for the subject (H) to know the state of the ultraviolet light quickly, based on light emitted from the light emitter (30).

The light emitter (30) notifies the subject (H) of the irradiation region (E1) of the ultraviolet light. It is thus possible for the subject (H) to know where the irradiation region (E1) is formed. If the subject (H) is exposed excessively to ultraviolet light, the health of the subject (H) may be impaired. The subject (H) can avoid such a situation by grasping the position of the irradiation region (E1). The active movement of the subject (H) to the irradiation region (E1) enables effective sterilization of the subject (H) through ultraviolet light.

The light emitter (30) notifies the subject (H) of the intensity of the ultraviolet light. Thus, the subject (H) can adjust the time of being exposed to the ultraviolet light, while taking the intensity of the ultraviolet light into consideration.

The light emitter (30) notifies the subject (H) of the wavelength of the ultraviolet light. Thus, the subject (H) can adjust the time of being exposed to the ultraviolet light, while taking the wavelength of the ultraviolet light into consideration.

The light emitter (30) notifies the subject (H) of the irradiation amount of the ultraviolet light. Thus, the subject (H) can adjust the time of being exposed to the ultraviolet light, while taking the irradiation amount of the ultraviolet light into consideration.

The light emitter (30) emits visible light such that the visible light overlaps with the irradiation region (E1) of the ultraviolet light emitted from the irradiator (20) (see FIG. 1). It is thus possible for the subject (H) to grasp the irradiation region (E1) of the ultraviolet light quickly and three-dimensionally.

The light emitter (30) is an LED that emits visible light. The subject (H) can be notified of the state of the ultraviolet light by a relatively simple configuration.

If the detector (40) detects the subject (H), the light emitter (30) notifies the subject (H) of the state of the ultraviolet light. Specifically, if the detector (40) detects the subject (H), the light emitter (30) is turned on. It is thus possible to reduce the chance of the light emitter (30) being turned on when the subject (H) is not present. This can prolong the life of the light emitter (30) and save energy consumption by the light emitter (30).

The ultraviolet light emitted from the irradiator (20) has a wavelength of from 190 nm or more to 280 nm or less. It is thus possible to obtain sufficient sterilization effect through ultraviolet light.

The ultraviolet light emitted from the irradiator (20) has a wavelength of from 190 nm or more to 230 nm or less. Setting the wavelength of the ultraviolet light at or below 230 nm can ease the influence of the ultraviolet light on the human health.

### «Second Embodiment»

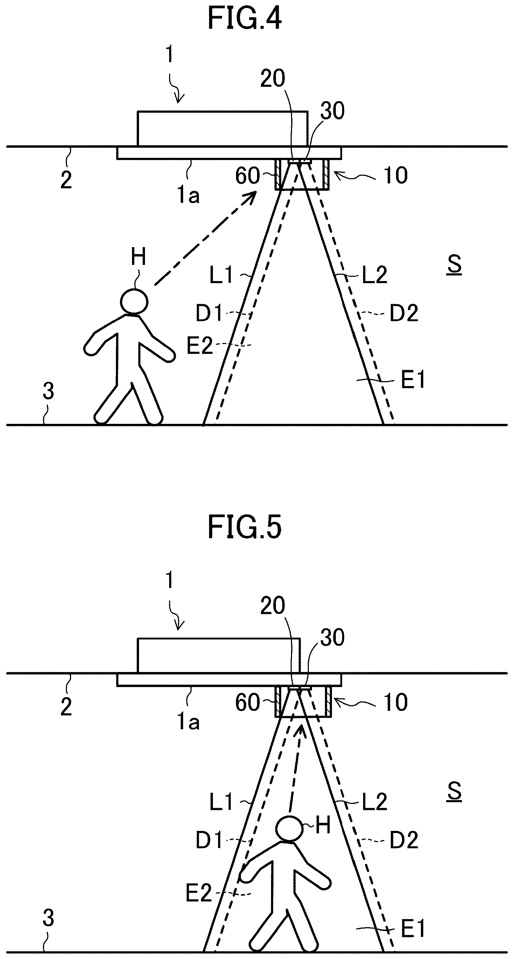

As illustrated in FIGS. 4 and 5, an ultraviolet light emitting device (10) according to a second embodiment includes a shielding member (60). The ultraviolet light emitting device (10) does not have to include a detector (40).

The shielding member (60) shields part of light emitted from the light emitter (30). The shielding member (60) is formed into a tubular shape surrounding the light emitter (30). The shielding member (60) may have a cylindrical shape, a rectangular cylindrical shape, or any other shape.

The shielding member (60) shields the light emitter (30) from the sight of the subject (H) when the subject (H) is outside the irradiation region (E1). The shielding member (60) does no shield the light emitter (30) from the sight of the subject (H) when the subject (H) is inside the irradiation region (E1). In other words, the shielding member (60) disallows the subject (H) outside the irradiation region (E1) to recognize the light emitter (30) visually and allows the subject (H) inside the irradiation region (E1) to recognize the light emitter (30) visually.

The controller (50) turns the light emitter (30) on when the irradiator (20) emits ultraviolet rays. As illustrated in FIG. 4, the shielding member (60) shields the light emitter (30) from the sight of the subject (H) when the subject (H) is outside the irradiation region (E1). The subject (H) can grasp that the subject (H) is outside the irradiation region (E1), based on the fact that the subject (H) cannot visually recognize the light emitter (30).

As illustrated in FIG. 5, the subject (H) can visually recognize the light emitter (30) when the subject (H) is inside the irradiation region (E1). The subject (H) can grasp that the subject (H) is inside the irradiation region (E1), based on the fact that the subject (H) can visually recognize the light emitter (30).

In addition, similarly to the first embodiment, the subject (H) can know the intensity, wavelength, or irradiation amount of the ultraviolet rays, based on the illuminance and hue of the light emitted from the light emitter (30).

Accordingly, it is possible to avoid the excessive exposure of the subject (H) to the ultraviolet rays. The active exposure of the subject (H) to the ultraviolet light enables effective sterilization.

### «Third Embodiment»

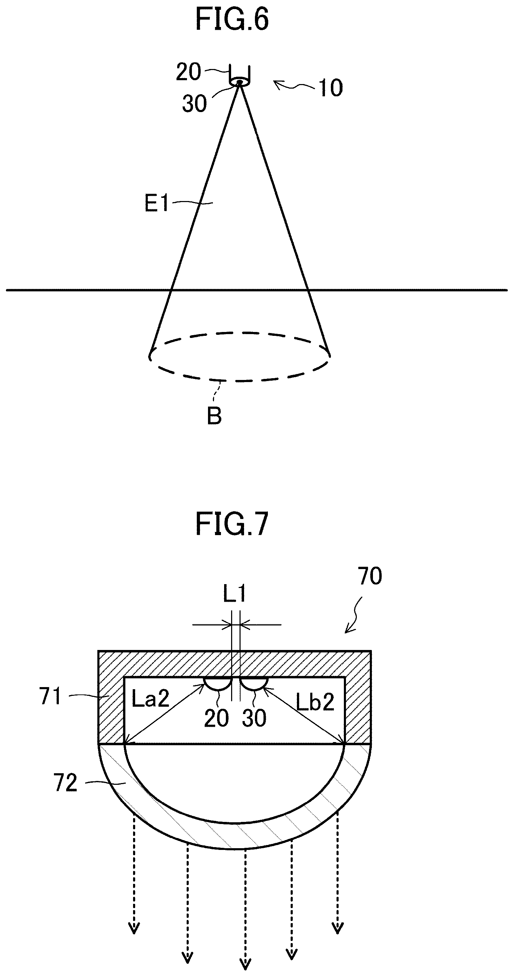

As illustrated in FIG. 6, an ultraviolet light emitting device (10) according to a third embodiment includes a light emitter (30) having a different configuration from the light emitters (30) of the first and second embodiments. The light emitter (30) of the third embodiment emits visible light such that the visible light indicates the boundary of the irradiation region (E1) of ultraviolet light emitted from the irradiator (20). Specifically, the light emitter (30) includes a semiconductor laser element and outputs colored light. The visible light (laser light) emitted from the light emitter (30) is formed along the irradiation region (E1).

Specifically, the light emitter (30) of the third embodiment emits visible light such that a circular boundary line (B) (the dashed line in FIG. 6) is projected onto the floor surface (3). The position of the boundary line (B) corresponds to the boundary of the irradiation region (E1) of the ultraviolet rays. The visible light emitted from the light emitter (30) has a red color, for example.

The subject (H) can know the irradiation region (E1) of the ultraviolet rays by visually recognizing the boundary line (B) on the floor surface (3). In addition, the subject (H) can know the intensity, wavelength, or irradiation amount of the ultraviolet rays, based on the illuminance and hue of light of the boundary line (B).

Accordingly, it is possible to avoid the excessive exposure of the subject (H) to the ultraviolet rays. The active exposure of the subject (H) to the ultraviolet light enables effective sterilization.

### «Fourth Embodiment»

In a fourth embodiment, an ultraviolet light emitting device (10) includes a lens (72) as a light guide member. As schematically illustrated in FIG. 7, the ultraviolet light emitting device (10) includes a housing (70). The housing (70) accommodates an irradiator (20) and a light emitter (30). The housing (70) includes a base (71) and the lens (72). The base (71) is recessed, and its bottom is provided with the irradiator (20) and the light emitter (30). The irradiator (20) and the light emitter (30) are arranged close to each other.

The lens (72) is attached to the base (71) to cover the opening of the base (71). The refractive index of the lens (72) is set so that the ultraviolet rays emitted from the irradiator (20) and the visible light emitted from the light emitter (30) are guided in the same direction.

Since the ultraviolet rays emitted from the irradiator (20) and the visible light emitted from the light emitter (30) are directed in the same direction (the direction indicated by the dashed arrows in FIG. 7), as described above, the subject (H) can localize the irradiation region (E1) of the ultraviolet rays, based on the visible light.

L1 represents the distance between the irradiator (20) and the light emitter (30); La2 represents the shortest distance between the irradiator (20) and the lens (72); Lb2 represents the shortest distance between the light emitter (30) and the lens (72); and L2 represents the average of the shortest distances La2 and Lb2. In the fourth embodiment, L1 is shorter than L2. Thus, the irradiator (20) and the light emitter (30) are close to each other, making it possible to reduce the chance of the ultraviolet rays and visible light being emitted in different directions. In this regard, it is preferable to cause the irradiator (20) and the light emitter (30) to be in contact with each other.

### «Fifth Embodiment»

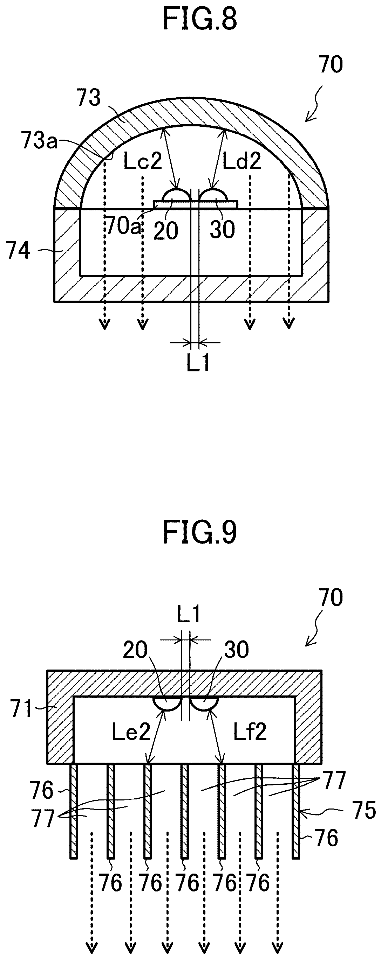

In a fifth embodiment, an ultraviolet light emitting device (10) includes a reflector (73) as a light guide member. As schematically illustrated in FIG. 8, the ultraviolet light emitting device (10) includes a housing (70). The housing (70) accommodates an irradiator (20) and a light emitter (30). The housing (70) includes the reflector (73) and a cover (74).

The reflector (73) is formed in the shape of a bowl having one open end. The reflector (73) is made of a metal material that reflects ultraviolet light and visible light. The irradiator (20) and the light emitter (30) are provided inside the reflector (73) via a support plate (70a). The irradiator (20) and the light emitter (30) are arranged close to each other. The irradiator (20) emits ultraviolet rays toward the inner surface (73a) of the reflector (73). The light emitter (30) emits visible light toward the inner surface (73a) of the reflector (73). The reflectance of the reflector (73) is set so that the ultraviolet rays emitted from the irradiator (20) and the visible light emitted from the light emitter (30) are guided in the same direction.

The cover (74) is attached to the reflector (73) to cover the open end of the reflector (73). The cover (74) is made of a translucent material through which light transmits. Instead of the cover (74), the lens (72) described in the fourth embodiment may be employed. When the ultraviolet light emitted from the irradiator (20) and the visible light emitted from the light emitter (30) are reflected off the reflector (73), the ultraviolet light and the visible light are directed in the same direction (the direction indicated by the dashed arrows in FIG. 8) and transmit through the cover (74).

Since the ultraviolet rays emitted from the irradiator (20) and the visible light emitted from the light emitter (30) are directed in the same direction, as described above, the subject (H) can localize the irradiation region (E1) of the ultraviolet rays, based on the visible light.

L1 represents the distance between the irradiator (20) and the light emitter (30); Lc2 represents the shortest distance between the irradiator (20) and the reflector (73); Ld2 represents the shortest distance between the light emitter (30) and the lens (72); and L2 represents the average of the shortest distances Lc2 and Ld2. In the fourth embodiment, L1 is shorter than L2. Thus, the irradiator (20) and the light emitter (30) are close to each other, making it possible to reduce the chance of the ultraviolet rays and visible light being emitted in different directions. In this regard, it is preferable to cause the irradiator (20) and the light emitter (30) to be in contact with each other.

### «Sixth Embodiment»

In a sixth embodiment, an ultraviolet light emitting device (10) includes a light blocking member (75) as a light guide member. As schematically illustrated in FIG. 9, the ultraviolet light emitting device (10) includes a housing (70). The housing (70) accommodates an irradiator (20) and a light emitter (30). The housing (70) includes a base (71) and the light blocking member (75). The light blocking member (75) includes a plurality of louvers (76). Slits (77) serving as holes through which ultraviolet light and visible light transmit are formed between the louvers (76). The base (71) is recessed, and its bottom is provided with the irradiator (20) and the light emitter (30). The irradiator (20) and the light emitter (30) are arranged close to each other.

The light blocking member (75) is attached to the base (71) to cover the opening of the base (71). The number of the louvers (76), the shape of each louver (76), and the spacing between adjacent louvers (76) of the translucent member (72) are set so that the ultraviolet rays emitted from the irradiator (20) and the visible light emitted from the light emitter (30) are guided in the same direction.

Since the ultraviolet rays emitted from the irradiator (20) and the visible light emitted from the light emitter (30) are directed in the same direction (the direction indicated by the dashed arrows in FIG. 9), as described above, the subject (H) can localize the irradiation region (E1) of the ultraviolet rays, based on the visible light.

L1 represents the distance between the irradiator (20) and the light emitter (30); Le2 represents the shortest distance between the irradiator (20) and the light blocking member (75) (strictly speaking, one of the louvers (76)); Lf2 represents the shortest distance between the light emitter (30) and the lens (72); and L2 represents the average of the shortest distances Le2 and Lf2. In the sixth embodiment, L1 is shorter than L2. Thus, the irradiator (20) and the light emitter (30) are close to each other, making it possible to reduce the chance of the ultraviolet rays and visible light being emitted in different directions. In this regard, it is preferable to cause the irradiator (20) and the light emitter (30) to be in contact with each other.

### «Variations»

Configurations such as those in the following variations may be employed in the above-described embodiments.

### -First Variation-

(1-A) The light emitter (30) may change the illuminance and hue in accordance with the irradiation time of the ultraviolet light emitted from the irradiator (20). The light emitter (30) reduces the illuminance of the visible light as the irradiation time passes. The light emitter (30) may turn off the light when the irradiation time reaches a predetermined time.

(1-B) The light emitter (30) may flash the visible light emitted from the light emitter (30) when the irradiator (20) is switched to the off-state after the elapse of a predetermined time since the irradiator (20) was turned on. It is thus possible for the subject (H) to know the end of the sterilization and disinfection by the irradiator (20) from the flashing visible light.

### -Second Variation- (not according to the claimed invention)

The recognizer may be a display.

(2-A) The display notifies the subject (H) of the state of the ultraviolet rays (such as the intensity, wavelength, irradiation amount, and irradiation region) using letters, symbols, icons, and other elements.

(2-B) In addition, the display may notify the subject (H) that the irradiator (20) is in the on-state. In this case, the display shows letters such as "Disinfecting" to notify the subject (H) of the state. The letters "Disinfecting" may flash on the display. This makes it easier for the subject (H) to know that the irradiator (20) is in the on-state.

(2-C) The display may indicate the intensity, wavelength, or irradiation amount of the ultraviolet rays numerically. The display may indicate the irradiation time of the ultraviolet rays numerically. The irradiation time of the ultraviolet rays may be a continuous irradiation time or a summation of irradiation times during a certain period (e.g., one day). The display may indicate letters "n minutes left until completion of disinfection" (n = 1, 2, ...).

### -Third Variation- (not according to the claimed invention)

The recognizer may be a sound producer that emits sound.

(3-A) The sound producer may emit sound from a speaker or any other similar component to notify the subject (H) of the state of the ultraviolet rays (such as the intensity, wavelength, irradiation amount, and irradiation region).

(3-B) The sound producer may notify the subject (H), by voice, that the subject (H) is in the irradiation region (E1) when the subject (H) enters the irradiation region (E1) of the ultraviolet rays. The above-described detector (40) detects whether the subject (H) is in the irradiation region (E1).

(3-C) The sound producer may change the volume of sound (an alarm) in accordance with the distance between the subject (H) and the irradiation region (E1). For example, as the subject (H) approaches the irradiation region (E1), the sound producer increases the volume of the sound (alarm). The above-described detector (40) detects the distance between the subject (H) and the irradiation region (E1).

(3-D) The sound producer may emit an alarm in accordance with the time in which the subject (H) is present in the irradiation region (E1). Specifically, if the time in which the subject (H) is present in the irradiation region (E1) exceeds a predetermined time, the sound producer emits an alarm. It is thus possible for the subject (H) to know that the subject (H) is exposed to the ultraviolet rays for a long time.

(3-E) The sound producer may notify the subject (H), by voice, of the irradiation time of the ultraviolet rays. The irradiation time of the ultraviolet rays may be a continuous irradiation time or a summation of irradiation times during a certain period (e.g., one day). The sound producer may make a sound "n minutes left until completion of disinfection" (n = 1, 2, . . .).

(3-F) The sound producer may be provided in a portable terminal owned by the subject (H). The portable terminal includes a cell phone, a smartphone, and a tablet terminal. Entry of the subject into the irradiation region (E1) may cause the portable terminal to make a sound. In this case, for example, wireless communications equipment, such as Bluetooth (registered trademark), may be used to detect the entry of the subject (H) in the irradiation region (E1).

(3-G) The sound producer may emit sound (an alarm) that can be heard by the subject (H) when the subject (H) enters the irradiation region (E1). In this case, the sound producer is disposed near the irradiation region (E1). The sound producer emits an alarm at a relatively low volume.

### -Fourth Variation- (not according to the claimed invention)

The recognizer may be an aroma generator configured to notify the subject (H), by an aroma, of the state of the ultraviolet rays. The aroma generator releases an aroma to notify the subject (H) of the state of the ultraviolet rays.

(4-A) The aroma generator may notify the subject (H) of the state of the ultraviolet rays (such as the intensity, wavelength, irradiation amount, and irradiation region) in accordance with the intensity of the aroma generated.

(4-B) The aroma generator may notify the subject (H), by an aroma, that the subject (H) is in the irradiation region when the subject (H) enters the irradiation region (E1) of the ultraviolet rays. The above-described detector (40) detects whether the subject (H) is in the irradiation region (E1).

(4-C) The aroma generator may change the intensity of an aroma in accordance with the distance between the subject (H) and the irradiation region (E1). For example, as the subject (H) approaches the irradiation region (E1), the aroma generator increases the intensity of the aroma. The above-described detector (40) detects the distance between the subject (H) and the irradiation region (E1).

(4-D) The aroma generator may release an aroma in accordance with the time in which the subject (H) is present in the irradiation region (E1). Specifically, if the time in which the subject (H) is present in the irradiation region (E1) exceeds a predetermined time, the aroma generator releases an aroma. It is thus possible for the subject (H) to know that the subject (H) is exposed to the ultraviolet rays for a long time.

(4-E) The aroma generator may release an aroma when the irradiator (20) is switched to the off-state after the elapse of a predetermined time since the irradiator (20) was turned on. It is thus possible for the subject (H) to know the end of the sterilization and disinfection by the irradiator (20) from the aroma.

### -Fifth Variation- (not according to the claimed invention)

The recognizer may be an air blower configured to blow wind to the subject (H) to notify the subject (H) of the state of the ultraviolet rays.

(5-A) The air blower may notify the subject (H) of the state of the ultraviolet rays (such as the intensity, wavelength, irradiation amount, and irradiation region) in accordance with the strength of the wind.

(5-B) The air blower may blow wind to the subject (H) when the subject (H) enters the irradiation region (E1) of the ultraviolet rays. This enables the subject (H) to know that the subject (H) is in the irradiation region (E1). The above-described detector (40) detects whether the subject (H) is in the irradiation region (E1).

(5-C) The air blower may change the strength of the wind in accordance with the distance between the subject (H) and the irradiation region (E1). For example, as the subject (H) approaches the irradiation region (E1), the air blower increases the strength of the wind. The above-described detector (40) detects the distance between the subject (H) and the irradiation region (E1).

(5-D) The air blower may blow wind to the subject (H) in accordance with the time in which the subject (H) is present in the irradiation region (E1). Specifically, if the time in which the subject (H) is present in the irradiation region (E1) exceeds a predetermined time, the air blower sends wind to the subject (H). It is thus possible for the subject (H) to know that the subject (H) is exposed to the ultraviolet rays for a long time.

(5-E) A fan of the air conditioner (1) may also serve as the air blower.

### -Sixth Variation-

The recognizer may be a temperature regulator configured to change the temperature of the air in the target space (S).

(6-A) The temperature regulator may change the temperature of the air of the subject (H) to notify the subject (H) of the state of the ultraviolet rays (such as the intensity, wavelength, irradiation amount, and irradiation region).

(6-B) A heat exchanger provided in the air conditioner (1) may also serve as the temperature regulator.

### «Other Embodiments»

The embodiments and variations described above may be implemented as follows.

The ultraviolet light emitting device (10) may include a means configured to change the irradiation region (E1) of the ultraviolet rays emitted from the irradiator (20). Examples of the means include a moving part, a shield, and a control circuit. The moving part changes the position and orientation of the light source of the irradiator (20). The shielding member (60) physically shields part of the irradiation region of the irradiator (20). The control circuit controls the irradiator (20) to enlarge or shrink the irradiation region (E1). The recognizer notifies the subject (H) of where in the target space (S) the changeable irradiation region (E1) is located.

The ultraviolet light emitting device (10) may be provided on a front surface of a wall-mounted air conditioner.

The air treating device may be an air cleaner, a dehumidifier, a humidifier, a ventilator, or any other similar device.

The ultraviolet light emitting device (10) does not necessarily have to be provided for the air treating device. The ultraviolet light emitting device (10) may be independently provided on the ceiling or a wall of a building. The ultraviolet light emitting device (10) may be provided in a mobile object, such as a vehicle or a train.

The ordinal numbers such as "first," "second," "third," .. . , described above are used to distinguish the terms to which these expressions are given, and do not limit the number and order of the terms.

### INDUSTRIAL APPLICABILITY

As can be seen from the foregoing description, the present disclosure is useful for an ultraviolet light emitting device and an air treating device.

### DESCRIPTION OF REFERENCE CHARACTERS

- 10: Ultraviolet Light Emitting Device
- 20: Irradiator
- 30: Light Emitter (Recognizer)
- 40: Detector
- 60: Shielding Member
- 72: Lens (Light Guide Member)
- 73: Reflector (Light Guide Member)
- 75: Light Blocking Member (Light Guide Member)
- 77: Slit (Hole)

## Claims

1. An ultraviolet light emitting device, comprising:
an irradiator (20) configured to emit ultraviolet light into a target space (S) where a subject is present; and
a recognizer (30) configured to notify the subject of a state of the ultraviolet light emitted from the irradiator (20),
wherein
the state of the ultraviolet light includes an intensity, an irradiation amount, or a wavelength of the ultraviolet light and wherein the state of the ultraviolet rays does not include an ON/OFF state of the irradiator (20), and
wherein
the recognizer includes a light emitter (30) configured to emit visible light,
the ultraviolet light emitting device further comprising:
a controller (50) configured to control the irradiator (20) and the light emitter (30), wherein
the controller (50) is configured to
control the intensity of the ultraviolet light emitted from the irradiator (20) and to change an illuminance or a hue of the visible light emitted from the light emitter (30) in accordance with the intensity of the ultraviolet light such that the subject can know the intensity of the ultraviolet rays emitted from the irradiator (20), based on the illuminance or hue of the visible light from the light emitter (30), or
control the irradiation amount of the ultraviolet light emitted from the irradiator (20) and to change the illuminance or the hue of the visible light emitted from the light emitter (30) in accordance with the irradiation amount of the ultraviolet light such that the subject can know the irradiation amount of the ultraviolet rays emitted from the irradiator (20), based on the illuminance or hue of the visible light from the light emitter (30), or
control the wavelength of the ultraviolet light emitted from the irradiator (20) and to change the illuminance or the hue of the visible light emitted from the light emitter (30) in accordance with the wavelength of the ultraviolet light such that the subject can know the wavelength of the ultraviolet rays emitted from the irradiator (20), based on the illuminance or hue of the visible light from the light emitter (30).

2. The ultraviolet light emitting device of claim 1, wherein
the light emitter (30) emits the visible light such that the visible light overlaps with the irradiation region of the ultraviolet light emitted from the irradiator (20).

3. The ultraviolet light emitting device of claim 1 or claim 2, wherein
the light emitter (30) emits the visible light such that the visible light indicates a boundary of the irradiation region of the ultraviolet light emitted from the irradiator (20).

4. The ultraviolet light emitting device of any one of claims 1 to 3, further comprising:
a shielding member (60) configured to shield the light emitter (30) from a sight of the subject outside the irradiation region of the ultraviolet light emitted from the irradiator (20).

5. The ultraviolet light emitting device of any one of claims 1 to 4, further comprising:
a light guide member (72, 73, 75) configured to guide the ultraviolet light emitted from the irradiator (20) and the visible light emitted from the light emitter (30) in a same direction.

6. The ultraviolet light emitting device of claim 5, wherein
a distance L1 between the irradiator (20) and the light emitter (30) is shorter than an average L2 of a shortest distance between the irradiator (20) and the light guide member (72, 73, 75) and a shortest distance between the light emitter (30) and the light guide member (72, 73, 75).

7. The ultraviolet light emitting device of claim 5 or 6, wherein
the light guide member (72, 73, 75) includes a lens (72) configured to change a direction of the ultraviolet light and a direction of the visible light.

8. The ultraviolet light emitting device of any one of claims 5 to 7, wherein
the light guide member (72, 73, 75) includes a reflector (73) configured to reflect the ultraviolet light and the visible light.

9. The ultraviolet light emitting device of any one of claims 5 to 8, wherein
the light guide member (72, 73, 75) includes a light blocking member (75) having a hole (77) through which the ultraviolet light and the visible light pass.

10. The ultraviolet light emitting device of any one of claims 1 to 9, wherein
the light emitter (30) is an LED.

11. The ultraviolet light emitting device of any one of claims 1 to 10, further comprising:
a detector (40) configured to detect the subject, wherein
if the detector (40) detects the subject, the recognizer (30) is configured to notify the subject of the state of the ultraviolet light.

12. The ultraviolet light emitting device of any one of claims 1 to 11, wherein
the ultraviolet light emitted from the irradiator (20) has a wavelength of from 190 nm or more to 280 nm or less.

13. The ultraviolet light emitting device of claim 12, wherein
the ultraviolet light emitted from the irradiator (20) has a wavelength of from 190 nm or more to 230 nm or less.

14. An air treating device, comprising:
the ultraviolet light emitting device (10) of any one of claims 1 to 13.

## Patentansprüche

1. Ultraviolettlicht emittierende Vorrichtung, umfassend:
eine Bestrahlungsvorrichtung (20), die so konfiguriert ist, dass sie Ultraviolettlicht in einen Zielraum (S) emittiert, in dem sich eine Person befindet; und
eine Erkennungsvorrichtung (30), die so konfiguriert ist, dass sie die Person über einen Zustand des von der Bestrahlungsvorrichtung (20) emittierten Ultraviolettlichts benachrichtigt, wobei
der Zustand des Ultraviolettlichts eine Intensität, eine Bestrahlungsmenge oder eine Wellenlänge des Ultraviolettlichts einschließt und wobei der Zustand der ultravioletten Strahlen keinen EIN/AUS-Zustand der Bestrahlungsvorrichtung (20) einschließt, und wobei
die Erkennungsvorrichtung einen Lichtemitter (30) einschließt, der so konfiguriert ist, dass er sichtbares Licht emittiert,
wobei die Ultraviolettlicht emittierende Vorrichtung weiter umfasst:
eine Steuereinheit (50), die so konfiguriert ist, dass sie die Bestrahlungsvorrichtung (20) und den Lichtemitter (30) steuert, wobei die Steuereinheit (50) so konfiguriert ist, dass sie
die Intensität des von der Bestrahlungsvorrichtung (20) emittierten Ultraviolettlichts steuert und eine Beleuchtungsstärke oder einen Farbton des von dem Lichtemitter (30) emittierten sichtbaren Lichts in Übereinstimmung mit der Intensität des Ultraviolettlichts so ändert, dass die Person die Intensität der von der Bestrahlungsvorrichtung (20) emittierten ultravioletten Strahlen erkennen kann, basierend auf der Beleuchtungsstärke oder dem Farbton des sichtbaren Lichts von dem Lichtemitter (30), oder
die Bestrahlungsmenge des von der Bestrahlungsvorrichtung (20) emittierten Ultraviolettlichts steuert und die Beleuchtungsstärke oder den Farbton des von dem Lichtemitter (30) emittierten sichtbaren Lichts in Übereinstimmung mit der Bestrahlungsmenge des Ultraviolettlichts so steuert, dass die Person die Bestrahlungsmenge der von der Bestrahlungsvorrichtung (20) emittierten ultravioletten Strahlen basierend auf der Beleuchtungsstärke oder dem Farbton des sichtbaren Lichts von dem Lichtemitter (30) erkennen kann, oder
die Wellenlänge des von der Bestrahlungsvorrichtung (20) emittierten Ultraviolettlichts steuert und die Beleuchtungsstärke oder den Farbton des von dem Lichtemitter (30) emittierten sichtbaren Lichts in Übereinstimmung mit der Wellenlänge des Ultraviolettlichts so ändert, dass die Person die Wellenlänge der von der Bestrahlungsvorrichtung (20) emittierten ultravioletten Strahlen basierend auf der Beleuchtungsstärke oder dem Farbton des sichtbaren Lichts von dem Lichtemitter (30) erkennen kann.

2. Ultraviolettlicht emittierende Vorrichtung nach Anspruch 1, wobei
der Lichtemitter (30) das sichtbare Licht so emittiert, dass das sichtbare Licht den Bestrahlungsbereich des von der Bestrahlungsvorrichtung (20) emittierten Ultraviolettlichts überlappt.

3. Ultraviolettlicht emittierende Vorrichtung nach Anspruch 1 oder Anspruch 2, wobei
der Lichtemitter (30) das sichtbare Licht so emittiert, dass das sichtbare Licht eine Grenze des Bestrahlungsbereichs des von der Bestrahlungsvorrichtung (20) emittierten Ultraviolettlichts anzeigt.

4. Ultraviolettlicht emittierende Vorrichtung nach einem der Ansprüche 1 bis 3, weiter umfassend:
ein Abschirmelement (60), das so konfiguriert ist, dass es den Lichtemitter (30) vor einem Blick der Person außerhalb des Bestrahlungsbereichs des von der Bestrahlungsvorrichtung (20) emittierten Ultraviolettlichts abschirmt.

5. Ultraviolettlicht emittierende Vorrichtung nach einem der Ansprüche 1 bis 4, weiter umfassend:
ein Lichtleitelement (72, 73, 75), das so konfiguriert ist, dass es das von der Bestrahlungsvorrichtung (20) emittierte Ultraviolettlicht und das von dem Lichtemitter (30) emittierte sichtbare Licht in dieselbe Richtung leitet.

6. Ultraviolettlicht emittierende Vorrichtung nach Anspruch 5, wobei
ein Abstand L1 zwischen der Bestrahlungsvorrichtung (20) und dem Lichtemitter (30) kürzer ist als ein Durchschnitt L2 eines kürzesten Abstands zwischen der Bestrahlungsvorrichtung (20) und dem Lichtleitelement (72, 73, 75) und eines kürzesten Abstands zwischen dem Lichtemitter (30) und dem Lichtleitelement (72, 73, 75).

7. Ultraviolettlicht emittierende Vorrichtung nach Anspruch 5 oder 6, wobei
das Lichtleitelement (72, 73, 75) eine Linse (72) einschließt, die so konfiguriert ist, dass sie eine Richtung des Ultraviolettlichts und eine Richtung des sichtbaren Lichts ändert.

8. Ultraviolettlicht emittierende Vorrichtung nach einem der Ansprüche 5 bis 7, wobei
das Lichtleitelement (72, 73, 75) einen Reflektor (73) einschließt, der so konfiguriert ist, dass er das Ultraviolettlicht und das sichtbare Licht reflektiert.

9. Ultraviolettlicht emittierende Vorrichtung nach einem der Ansprüche 5 bis 8, wobei
das Lichtleitelement (72, 73, 75) ein Lichtblockierelement (75) mit einem Loch (77) einschließt, durch das das Ultraviolettlicht und das sichtbare Licht hindurchtreten.

10. Ultraviolettlicht emittierende Vorrichtung nach einem der Ansprüche 1 bis 9, wobei der Lichtemitter (30) eine LED ist.

11. Ultraviolettlicht emittierende Vorrichtung nach einem der Ansprüche 1 bis 10, weiter umfassend:
einen Detektor (40), der so konfiguriert ist, dass er die Person detektiert, wobei
wenn der Detektor (40) die Person detektiert, die Erkennungsvorrichtung (30) so konfiguriert ist, dass sie die Person über den Zustand des Ultraviolettlichts benachrichtigt.

12. Ultraviolettlicht emittierende Vorrichtung nach einem der Ansprüche 1 bis 11, wobei
das von der Bestrahlungsvorrichtung (20) emittierte Ultraviolettlicht eine Wellenlänge von 190 nm oder mehr bis 280 nm oder weniger aufweist.

13. Ultraviolettlicht emittierende Vorrichtung nach Anspruch 12, wobei
das von der Bestrahlungsvorrichtung (20) emittierte Ultraviolettlicht eine Wellenlänge von 190 nm oder mehr bis 230 nm oder weniger aufweist.

14. Luftbehandlungsvorrichtung, umfassend:
die Ultraviolettlicht emittierende Vorrichtung (10) nach einem der Ansprüche 1 bis 13.

## Revendications

1. Dispositif d'émission de lumière ultraviolette, comprenant :
un irradiateur (20) configuré pour émettre de la lumière ultraviolette dans un espace cible (S) où un sujet est présent ; et
un reconnaisseur (30) configuré pour informer le sujet d'un état de la lumière ultraviolette émise par l'irradiateur (20), dans lequel
l'état de la lumière ultraviolette inclut une intensité, une quantité d'irradiation ou une longueur d'onde de la lumière ultraviolette et dans lequel l'état des rayons ultraviolets n'inclut pas un état MARCHE/ARRÊT de l'irradiateur (20), et
dans lequel
le reconnaisseur inclut un émetteur (30) de lumière configuré pour émettre une lumière visible,
le dispositif d'émission de lumière ultraviolette comprenant en outre : un dispositif (50) de commande configuré pour commander l'irradiateur (20) et l'émetteur (30) de lumière, dans lequel le dispositif (50) de commande est configuré pour
commander l'intensité de la lumière ultraviolette émise par l'irradiateur (20) et pour modifier un éclairement ou une teinte de la lumière visible émise par l'émetteur (30) de lumière en fonction de l'intensité de la lumière ultraviolette de sorte que le sujet puisse connaître l'intensité des rayons ultraviolets émis par l'irradiateur (20), sur la base de l'éclairement ou de la teinte de la lumière visible provenant de l'émetteur (30) de lumière, ou
commander la quantité d'irradiation de la lumière ultraviolette émise par l'irradiateur (20) et modifier l'éclairement ou la teinte de la lumière visible émise par l'émetteur (30) de lumière en fonction de la quantité d'irradiation de la lumière ultraviolette de sorte que le sujet puisse connaître la quantité d'irradiation des rayons ultraviolets émis par l'irradiateur (20), sur la base de l'éclairement ou de la teinte de la lumière visible provenant de l'émetteur (30) de lumière, ou
commander la longueur d'onde de la lumière ultraviolette émise par l'irradiateur (20) et modifier l'éclairement ou la teinte de la lumière visible émise par l'émetteur (30) de lumière en fonction de la longueur d'onde de la lumière ultraviolette de sorte que le sujet puisse connaître la longueur d'onde des rayons ultraviolets émis par l'irradiateur (20), sur la base de l'éclairement ou de la teinte de la lumière visible provenant de l'émetteur (30) de lumière.

2. Dispositif d'émission de lumière ultraviolette selon la revendication 1, dans lequel
l'émetteur (30) de lumière émet la lumière visible de sorte que la lumière visible chevauche la région d'irradiation de la lumière ultraviolette émise par l'irradiateur (20).

3. Dispositif d'émission de lumière ultraviolette selon la revendication 1 ou la revendication 2, dans lequel
l'émetteur (30) de lumière émet la lumière visible de sorte que la lumière visible indique une limite de la région d'irradiation de la lumière ultraviolette émise par l'irradiateur (20).

4. Dispositif d'émission de lumière ultraviolette selon l'une quelconque des revendications 1 à 3, comprenant en outre :
un organe de protection (60) configuré pour protéger l'émetteur (30) de lumière d'une vue du sujet en dehors de la région d'irradiation de la lumière ultraviolette émise par l'irradiateur (20).

5. Dispositif d'émission de lumière ultraviolette selon l'une quelconque des revendications 1 à 4, comprenant en outre :
un organe (72, 73, 75) de guidage de lumière configuré pour guider la lumière ultraviolette émise par l'irradiateur (20) et la lumière visible émise par l'émetteur (30) de lumière dans une même direction.

6. Dispositif d'émission de lumière ultraviolette selon la revendication 5, dans lequel
une distance L1 entre l'irradiateur (20) et l'émetteur (30) de lumière est plus courte qu'une moyenne L2 d'une distance la plus courte entre l'irradiateur (20) et l'organe (72, 73, 75) de guidage de lumière et d'une distance la plus courte entre l'émetteur (30) de lumière et l'organe (72, 73, 75) de guidage de lumière.

7. Dispositif d'émission de lumière ultraviolette selon la revendication 5 ou la revendication 6, dans lequel
l'organe (72, 73, 75) de guidage de lumière inclut une lentille (72) configurée pour modifier une direction de la lumière ultraviolette et une direction de la lumière visible.

8. Dispositif d'émission de lumière ultraviolette selon l'une quelconque des revendications 5 à 7, dans lequel
l'organe (72, 73, 75) de guidage de lumière inclut un réflecteur (73) configuré pour réfléchir la lumière ultraviolette et la lumière visible.

9. Dispositif d'émission de lumière ultraviolette selon l'une quelconque des revendications 5 à 8, dans lequel
l'organe (72, 73, 75) de guidage de lumière inclut un organe (75) de blocage de lumière présentant un trou (77) à travers lequel passent la lumière ultraviolette et la lumière visible.

10. Dispositif d'émission de lumière ultraviolette selon l'une quelconque des revendications 1 à 9, dans lequel l'émetteur (30) de lumière est une DEL.

11. Dispositif d'émission de lumière ultraviolette selon l'une quelconque des revendications 1 à 10, comprenant en outre :
un détecteur (40) configuré pour détecter le sujet, dans lequel
si le détecteur (40) détecte le sujet, le reconnaisseur (30) est configuré pour informer le sujet de l'état de la lumière ultraviolette.

12. Dispositif d'émission de lumière ultraviolette selon l'une quelconque des revendications 1 à 11, dans lequel
la lumière ultraviolette émise par l'irradiateur (20) a une longueur d'onde de 190 nm ou plus à 280 nm ou moins.

13. Dispositif d'émission de lumière ultraviolette selon la revendication 12, dans lequel
la lumière ultraviolette émise par l'irradiateur (20) a une longueur d'onde de 190 nm ou plus à 230 nm ou moins.

14. Dispositif de traitement d'air, comprenant :
le dispositif (10) d'émission de lumière ultraviolette selon l'une quelconque des revendications 1 à 13.
